# EUROPEAN PATENT APPLICATION

(11) **EP 1 946 734 A1**
(43) Date of publication of application: **23.07.2008**
(21) Application number: 07000723.2
(22) Date of filing: 15.01.2007
(51) Int. Cl.: A61F 13/511, B32B 3/24, B32B 27/20, B32B 27/32

(54) **Topsheet and method for its manufacture**

(71) Applicant: RKW AG Rheinische Kunststoffwerke, 67547 Worms (DE)
(72) Inventor: Teissier, Marie Pierre, 01480 Jassan Riottier (FR); Grefenstein, Achim, 67122 Altrip (DE); Loynet, Pierre, 69110 St Foy les Lyon (FR)
(74) Representative: Wagner, Jutta

(57) **Abstract**

The present invention provides multilayer films useful as topsheet with a top layer comprising a thermoplastic material and from 5 to 25 % of inert particles with a mean diameter of 1 to 5 µm and an aspect ratio of 1 to 5, as well as a method for manufacturing them comprising the steps of providing a multilayer precursor film, perforating it and embossing the perforated film at a temperature below 70 °C as well as absorbent articles containing the multilayer film as topsheet.

## Description

The present invention relates to multilayer films useful as topsheet and to a method for manufacturing them as well as to absorbent articles containing them.

Disposable absorbent articles are well known in the prior art and include such articles as diapers, bandages, catamenials, and the like. Such articles generally comprise a fluid permeable topsheet, a fluid impervious backsheet and an absorbent element in between the two. The topsheet overlays the absorbent element and forms the surface that is in contact with the wearer. The function of the topsheet imposes many necessary properties that partly contradict each other. A topsheet shall
- rapidly absorb liquid, i.e. pass it on into the absorbent element
- prevent absorbed liquid from re-wetting the surface
- provide a dry surface feeling
- have a pleasant, textile like feeling on the skin, i.e. be pliable and soft
- securely encase the absorbent element, i.e. be strong
- be thin and easy to manufacture in view of cost efficiency

Many topsheet variations are taught in the prior art such as fibrous webs which may be woven or non-woven (carded or spunbond) and perforated plastic films. There are numerous examples for all variations.

While fibrous webs are ideal from a standpoint of haptics, topsheets made from perforated plastic films have some advantages:
They are easy and thus also cost effective in production. The form of the perforations allows to regulate properties such as fastness of absorption into the absorbent element and re-wet upon applying pressure. Liquid will not rest on the surface. On the other hand, plastic films lack the soft, textile like touch of woven and non-woven webs. They have a sticky surface that is often perceived as stuffy. In order to improve the touch of topsheets from plastic films there are numerous approaches known.

The most common one is to texture the surface of the film or to impart a three-dimensional pattern by embossing. There are also proposals to combine a textured surface with a three-dimensional structure made by embossing. The size of the structures ranges from very small, microscopic structures to rather big structures perceivable with the naked eye.

One example is EP 018 684 proposing to provide microscopic nubbles on the surface of the film. The nubbles are made by a kind of embossing step using a roller to which particles with a mean diameter of 0,04 to 0,08 mm embedded in an epoxy resin are applied.

Another example is US 6,548,158 using a multilayer film with an outer layer containing 30 to 60 parts by weight of a filler with a particle size from 2 to 20 µm to roughen the surface.

JP 2001341218 tries to modify the surface by adding 30 to 80 % by weight of a filler that will form a sea-island structure with the base resin.

Still another example is described in EP 1 621 169 where an apertured film is provided with a multiplicity of surface structures and in EP 1 198 214 where a film with micro apertures and macro apertures is used.

However, none of the prior art approaches is ideal with respect to all necessary properties and there still is a need for further improved topsheet materials.

It has now been found that perforated multilayer films with a top layer comprising a thermoplastic material containing 5 to 25 % by weight of inert particles with a mean diameter around 3 µm provide very soft, cloth like topsheets that also fulfill the other requirements like fast absorption, dry surface, and sufficient mechanical stability for handling and during use.

Thus according to the present invention there are provided perforated multilayer films with a top layer comprising a thermoplastic material and from 5 to 25 % by weight of inert particles with a mean diameter of from 1 µm to 5 µm.

The top layer of the films according to the invention comprises one or more thermoplastic base materials like polyolefins. Preferably the thermoplastic material is a polyethylene and especially a low density polyethylene (LDPE), a linear low density polyethylene (LLDPE). The LLDPE may be one made with conventional catalysts like Ziegler-Natta catalysts or it may be made by Metallocene catalysts (m-LLDPE). It also possible and preferred to use a mixture of two or more of the said materials, i.e. a mixture of LDPE and LLDPE or a mixture of LDPE, LLDPE and m-LLDPE or a mixture of LLDPE and m-LLDPE.

The inert particles may be any kind of particles that retain their structure during manufacture of the film, that are acceptable for use in articles contacting the human skin and have the desired mean diameter. Suitable are for example mineralic fillers, preferred are calcium carbonate, glass beads and barium sulfate and especially preferred is calcium carbonate. The particles may be coated in a manner known as such, e.g. with fatty acid esters. The mean particle size is an important property and should range from 1 µm to 5 µm, preferably from 2 µm to 4 µm and especially preferred from 2,5 µm to 3,5 µm. The particle size is determined by sieving with sieves of different sizes. The mean particle size, also called d50%, is characterized by the fact that 50% of all particles have a size equal or smaller than that value. The size of the particles ranges preferably from 0,1 to 15 µm, preferably from 1 to 10 µm.

The aspect ratio of the particles should preferably range from 1 to 5, especially preferred from 1 to 2. Especially preferred are mineralic fillers with an aspect ratio (length/width-ratio in all three dimensions) close to 1, i.e. more or less spherical particles. The aspect ratio is determined by counting several (at least 10) particles photographed via REM-microscope and measuring their greatest length and smallest width. The aspect ratio is the quotient of length and width.

The amount of inert particles should be at least 5 % by weight of the top layer and preferably more than 10 % by weight to achieve a sufficient effect. On the other hand it is preferred to use 25 % by weight or less, and especially less than 20 % by weight to avoid a too high stiffness and hardness. Preferred amounts range from 11 to 20 % by weight and especially preferred are 12 to 17 % by weight.

As such it is to be noted that the amount is higher than that usually applied for purposes of coloring a film. In fact, in order to obtain a coloring the films according to the invention may additionally contain usual pigments, like e.g. up to 10 % by weight of TiO₂ for a white coloring. TiO₂ is, due to its high aspect ratio, not considered as inert particles according to this invention. The amount of particles is also lower than that usually applied for imparting e.g. breathability by stretching or good printability in non-stretched synthetic paper. Where a filler is used for imparting breathability, usually 50 %, 60 % by weight or even more filler is used and the precursor film made by blown or cast extrusion is then stretched. Upon stretching, microapertures form in the film that allow passage of water vapor but not of liquids. Since the films according to the invention are perforated and thus liquid permeable there is no need for stretching and providing microapertures. The amount of inert particles used according to the invention would also be insufficient for such a purpose.

The top layer according to the invention may contain further usual ingredients like additives, colorants and so on in their usual amounts. The top layer(s) may thus additionally comprise one or more of colorants, mineralic fillers, processing aids, antistatic agents, antislip agents, stabilizers, antioxidants, acid neutralizing agents, ultraviolet absorbers, antiblocking agents and antifogging agents. The additives are used in their usual amounts, typically up to 5 % additives, if colorants are present typically up to 15 % by weight additives are present.

The multilayer film comprises at least one further layer apart from the top layer. It may comprise one, two, three, four, five or even more additional layers, one or two additional layers being preferred, one additional layer being especially preferred.

The composition of the further layer is not specifically restricted. It is however preferred that the further layer is coextrudable or (less but still preferred) bonded via direct extrusion of one of the layers onto the other and thus the materials should be able to provide suitable adhesion with the top layer upon coextrusion or direct extrusion. In the case of two or more further layers, they can comprise the same or different components, usually they will differ in composition. It is also possible but not preferred to laminate a top layer with the further layer(s) by any means known in the art such as thermobonding or adhesives.

Suitable materials for the further layer(s) are thermoplastic materials like LDPE, LLDPE, mLLDPE, PP-Homo- or Copolymers. Preferred materials are the thermoplastic materials as described above for the top layer with or without inert particles, preferred without inert particles.

The further layer(s) may comprise usual ingredients like fillers, additives, colorants and so on in their usual amounts. Thus they may additionally comprise common additives like colorants, mineralic fillers, processing aids, antistatic agents, antislip agents, stabilizers, antioxidants, acid neutralizing agents, ultraviolet absorbers, antiblocking agents and antifogging agents. The additives are used in their usual amounts, typically up to 5 % additives, with colorants up to 15 % by weight, are present. The additives in the further layers may be the same or different and be present in the same or differing amounts in case more than one further layer is present.

In a first presently preferred embodiment the multilayer film has two layers with the top layer containing more than 10% by weight of the layer of inert particles, preferred 15%, 50 to 80% by weight of the layer of m-LLDPE, preferred 75%, and additives like antifogging agents and processing aids in an amount of 10%. The further layer contains 50 to 90% by weight of the layer of LDPE, preferred 75 %, 10 to 20% by weight of the layer of LLDPE, preferred 15% and additives like colorants, processing aids and antifogging agent with 10% by weight of the layer. Preferably, the thickness of the top layer is 5 µm and of the inner layer is 20 µm.

In a second presently preferred embodiment the film is a multi layer film with two layers wherein the top layer contains more than 10% by weight of the layer of inert particles, preferred 15%, 50 to 80% by weight of the layer of LDPE, preferred 75%, and additives like antifogging agents and processing aids in an amount of 10%. The further layer contains 50 to 90% by weight of the layer of LDPE, preferred 70 %, 10 to 30% by weight of the layer of LLDPE, preferred 20% and additives like colorants, processing aids and antifogging agent with 10% by weight of the layer. Preferably, the thickness of the top layer is 5 µm and of the inner layer is 20 µm

Suitable LDPE is commercially available and has a MFI (190 °C/ 2.16 kg) from 1.95 to 2.5 g/10 min, preferably 2 g/10 min and a melting point from 110 °C to 120 °C, preferably 116 °C. Suitable m-LLDPE is commercially available and has a MFI (190 °C/ 2.16 kg) from 2.5 to 3.5 g/10 min, preferably 3 g/10 min and a melting point of from 60 °C to 80 °C, preferably 68 °C. Suitable LLDPE is commercially available and has a MFI (190 °C/ 2.16 kg) from 4 to 5 g/10 min, preferably 4.4 g/10 min and a melting point from 110 °C to 130 °C, preferably 127 °C.

The thickness ratio of the top layer to the further layer(s) is preferably from 1:2 to 1:10, especially preferred from 1:3 to 1:5. The thickness of the films lies typically in the range of 15 to 50 µm, preferably 20 to 30 µm.

The films may be manufactured and perforated according to any method.

Typically a precursor film will be formed by blown film extrusion or cast extrusion of molten thermoplastic material containing any other desired component, whereby the material for the top layer additionally contains the inert particles. The precursor film may also preferably be textured and/or embossed upon extrusion.

The precursor film is then perforated, preferably by vacuum and application of hot air.

The film may further be stretched, ring rolled, printed, etc. after or before perforating. Stretching serves to reduce the thickness of the films and stretching and Ring-rolling improve the mechanical properties. Ring-rolling can also impart or improve elasticity of the film.

It has further been found that the surface feeling of films can be improved, i.e. the haptics can be made more similar to that of textiles, by cold embossing a perforated and textured film.

Thus, the present invention also provides a method for manufacturing perforated films suitable as topsheet comprising the steps of providing a multilayer precursor film, texturing the surface of the film, perforating the film and embossing the perforated film at a temperature from 15 to 70 °C.

With the method according to the invention the surface of the films becomes soft and textile like, while the liquid absorption and re-wet properties remain excellent.

The first step of the method according to the invention is manufacturing a precursor film whereby the surface of the film is textured e.g. via common embossed cast film rollers. This step is well known per se in the art.

The film may be a mono layer film from thermoplastic materials as described above for the top layer(s). Preferably the precursor film is a multilayer precursor film as described above, i.e. with a top layer or top layers from a filled thermoplastic material as described above.

The texturing may be of any pattern, preferred are for example diamond shaped cells. They may suitably have a width and length of 200 µm each, and a depth of 45 µm and are arranged in machine direction.

The film is then, preferably directly as a next step and especially preferred in-line, perforated by means known as such. It is also possible to store the film and perform the perforation at a later time (off-line).

Preferably perforation is accomplished by passing the heated film over an apertured roll having a vacuum applied from the inside. The film may be heated by passing it over heated rollers, by infrared radiation and/or by directing a stream of hot air onto the film. The thermoplastic material softens and the vacuum causes the film to be sucked into the apertures and finally rupture so that perforations result. The preferred process used is described in detail in US 4,151,240 issued to Lucas et al on April 24, 1979. The device includes a constant tension film supply before the perforation drum and constant tension forwarding and winding. To perforate the film there is a heating hot air jet against one surface of the film while applying vacuum adjacent the opposite surface of the film. For example, the film may be heated by means of hot air (350 - 550 °C) to a temperature between near the melting point (for PE around 110 °C and 130 °C) and sucking the hot web on a porous roll.

The structure of the apertures in the roll determines the structure of the perforations. The vertical profile, form and size of the apertures has to be adapted to provide perforation of the intended shape as is per se known in the art.

The vertical profile of the perforations, i.e. the form as viewed perpendicular to the film surface, influences the absorption and re-wet properties. Perforations with a diameter that is smaller on the side of the absorbing pad than on the body facing side will considerably lessen re-wet. Thus, it is preferred to use apertures providing conical or other non-uniform diameter perforations with respect to the vertical profile.

The outer contour or form may be of any kind. Mention may be made of simple, convex polygons, like rhombus, trapeze, quadrate, rectangle, pentagon, hexagon, octagon and so on, and structures with curved contours, like circles and ellipses. It is also possible to use forms combining straight lines and curves like rectangles with circular ends.

The size of the perforations should range from 0.1 to 10 mm², preferably from 0,2 to 7 mm² (0.5 to 3 mm diameter) and especially preferred around 3 mm². The mean distances between the perforations should be 0.1 to 1.0 mm, preferably approximately 0.4 mm. Obviously the actual distance will vary for some patterns like ellipses or circles and can for different directions with patterns like rhombus or hexagon. It is preferred to use approximately equal distances between the perforations in all directions, i.e. to arrange them evenly.

The pattern and/or arrangement influences the mechanical properties whereas the size of pattern influences both mechanical and hygiene properties.

Of course it is also possible to use other means of perforation, like needling or jets of liquid etc. that are known per se.

The perforation step may also be preceded by other steps like printing, stretching and so on.

The perforated film is then embossed at a temperature of up to 70 °C, preferably up to 60 °C and especially preferred up to 50 °C. The lower limit of the temperature depends on the material, for useful thermoplastic materials it lies between 10 °C and 20 °C, usually around 15 °C. It is important that this embossing is performed at comparably low temperatures. When the usual higher temperatures like 80 °C or more are used, the soft, textile like touch is not achieved. Furthermore, especially for perforation patterns with big hole size the strike-through properties will be reduced dramatically during hot embossing. This is due to the effect of closing the pores by melting material.

Generally speaking the bigger the perforation size, the lower embossing temperature is useful/needed and on other hand with small perforations a higher embossing temperature is possible. Within the range according to the invention there will generally be an optimum temperature with a view to haptic properties that can easily be determined with a few tests for a given material, perforating and embossing pattern. This temperature will not always be the optimum with a view to re-wet and strike through, also abbreviated as hygienic properties in the following. In such instances a compromise between optimizing haptics and acceptable hygienic properties has to be found by way of a few tests. The films manufactured according to the invention will however show superior haptic as compared to films manufactured according to the prior art.

The pattern of the embossing should differ from the texture applied directly after extruding the film. Otherwise, it is not specifically restricted. Preferred patters are lines, straight and curved, crossing lines, interrupted lines, dots, points and so on. Especially preferred are rectangular or other straight lines crossing each other.

Preferably the lines have a distance from each other of 100 to 800 µm, especially preferred 200 to 600 µm and most preferred 300 to 500 µm.

Before and after the embossing station there is usually a system to control the tension of the film. The film is driven between two rolls. One roll with a special embossing and the counter roll with a smooth surface which could be e.g. metal, rubber or paper. Both of them turn in opposite direction. In a preferred embodiment, the roll and the counter roll are manufactured from a rigid material and are mounted with an adjustable pressure between the two rolls. The embossed roll is temperature regulated and from metal.

As stated above, the film may additionally be printed, stretched, ring-rolled or subjected to any other known process, of course with the exception of embossing at high temperatures, calendaring and the like. In a preferred embodiment the film is not stretched or ring-rolled.

The films are ideally suited as topsheets for absorbent articles like catamenial pads, diapers, incontinence products and the like. Thus, the invention also relates to such articles comprising the multilayer films according to the invention as topsheet, whereby the top layer of the films forms the body-facing side of the topsheet.

The design and manufacture of absorbent articles is known as such and shall therefore not be described here. Reference may be made for example to the prior art cited in this application.

The invention will be illustrated by way of the following examples, which shall not be construed to limit the scope of the invention to the specific embodiments described. If not stated otherwise, all percentages and parts are given by weight.

### Examples

### Materials

The materials used are:
LDPE: MFI (190 °C, 2.16 kg) 2 g/min, Density 0.928 g/cm³
LLDPE: MFI (190 °C, 2.16 kg) 4.4 g/min, Density 0.936 g/cm³
m-LLDPE: MFI (190 °C, 2.16 kg) 3 g/min, Density 0.875 g/cm³
filler: Masterbatch MFI (190 °C, 2.16 kg) 9.1 g/min, density 1.58 g/cm³,
   containing 35 % LLDPE and 65 % CaCO₃,
color additive: Masterbatch MFI (190°C, 2.16 kg) 2.5 g/min, density 1.73 g/cm³
   containing at least 60% TiO₂
processing aid: ARX 741 (ARGUS)
antifogging additive: AF 1088 (Schulman)

From these materials the follwing mixtures (table 1) were prepared:

**Table 1**

| material | amount in mixt 1 | amount in mixt. 2 | |
|---|---|---|---|
| | | top layer | further layer |
| LDPE | 75 % | | 75 % |
| LLDPE | 10 % | | 15 % |
| m-LLDPE | 5 % | 75 % | |
| filler | | 15 % | |
| additives | 10 % | 10% | 10 |

As an additional comparison the commercially available topsheet film "Petalo Blue" from Procter & Gamble was used.

### Manufacturing of films

For the test precursor films have been extruded from mixtures 1 and 2 on a cast film line, perforated and, if applicable, embossed.

### Test methods

The films were evaluated by a test panel of 20 persons for their haptic properties. Samples of 6 x 13 cm were cut from the film, applied (glued) on carton and an acquisition distribution layer (ADL) out of a carded non woven with 36g/cm² in order to simulate the touch on a napkin. The films are presented to the panel in a random order in blind. Each tester accords a rating of softness on a scale from 1 to 14 points, whereby 14 points is very soft and 1 point corresponds to a low softness. The points accorded are summed up to give the result. The maximum is 280 points.

Further, strike-through and re-wet properties were measured according to edana test methods 150.5-02 and 151.3-02 as follows.

For both, a standard absorbent pad, consisting of five layers of reference filter paper (100 mm x 100 mm) and having a mean strike-through time without any cover of 3 ± 0.5 sec and a liquid absorption capacity of 480 ± 30 % is used. The test liquid is Plasmion gelatine solution (Fresenius Kabi, France).

The strike through is the time until 5 ml of the liquid are absorbed and is determined with the standard electrode positioned on the test sample which in turn is positioned on top of the absorbent pad. For hygienic applications a strike through of lower than 6 s, preferred lower 5 s, and especially preferred lower 4 s is desired.

For measuring the re-wet the following utensils are used: a pick-up paper (with 90 ± 4 g/m² and an air flow resistance (ISO 5636) of 1,9 ± 0.3 kPa) and a simulated baby weight made from a 10 x 10 cm stainless steel base of 4000 ± 20 g, a polyurethane foam rubber wrapped into polyethylene film of 25 µm thickness and fixed to the steel base with adhesive tape. The re-wet is measured by absorbing liquid with the loading factor indicated on the cover of the box of. into the pad with the sample on top, putting the baby weight onto the sample and pad for 3 minutes, removing residual liquid from the surface and placing weighed pick-up paper on the top. Then the baby weight is again placed on top for 2 minutes and afterwards the pick-up paper is weighed and the re-wet noted as weight difference of the pick-up paper before and after the test. The re-wet for hygienic applications should be lower than 0.5 g, preferably lower than 0.2 g and especially preferred lower than 0.1 g.

For each test the mean value of 6 samples is given. All tests have been performed at 23 °C and with 70 % relative humidity.

### Example 1

One and two layer films were made from mixture 1 and mixture 2, respectively, with approximately rhombic perforations (length 2050 µm, width 945 µm). The distance between perforations is 500 µm. The films were not embossed. The' results for the haptic test, re-wet and strike through are summarized in table 2. "Petalo Blue" was used as an additional comparison.

**Table 2**

| Designation | material | Rating touch | Re-wet | Strike through |
|---|---|---|---|---|
| 1a | mixt. 1 | 224 | 0.047 g | 3.2 s |
| 1b (Comp.) | mixt. 2 | 129 | 0.052 g | 3.07 s |
| Petalo Blue (Comp.) | | 127 | 0.45 g | 3.4 s |

The use of the top layer according to the invention provides topsheets with superior touch and very good re-wet and strike through characteristics. If a material according to the prior art is used for the top layer the re-wet and strike through are also very good but the haptic is not good. The commercial film is worse with respect to all properties, whereby the re-wet is outside the desired range.

### Example 2

One and two layer films were made from mixture 1 and mixture 2, respectively, with elliptic perforations (length 950 µm, width 400 µm, radius 200µm). The distance in length and width is approximately 250 µm. The films were either not embossed, embossed at 35 °C and 60 °C. The results for the haptic test, re-wet and strike through are summarized in table 3. Again, the commercial film "Petalo Blue" was used as additional comparison.

**Table 3**

| designation | Material | Embossing + temper. | Rating touch | Re-wet | Strike through |
|---|---|---|---|---|---|
| 2a | mixt. 2 | No | 131 | 0.073 g | 3.08 s |
| 2b (Comp.) | mixt. 1 | No | 80 | 0.077 g | 3.5 s |
| 2c | mixt. 2 | yes, 35 °C | 131 | 0.077 g | 4.28 s |
| 2d | mixt. 2 | yes, 60 °C | 173 | 0.474 g | 5.64 s |
| Petalo Blue (Comp.) | | | 127 | 0.45 | 3.4 |

With this perforation pattern the haptic properties of the film are improved by the top layer according to the invention as compared to a usual material and are better than for the commercial film but are still not optimal. With the method according to the invention haptic can be improved dramatically with only slight losses in re-wet and strike through. Thus, the combination of the material and method provided topsheets with balanced properties that are obtained at competitive costs.

### Example 3

Two layer films were made according to mixture 2 with approximately diamond shaped perforations (length 790 µm, width 613 µm). The distance in length and width is approximately 420 µm. The films were embossed at either 35 °C, 60 °C or 80 °C. The results for the haptic test, re-wet and strike through for a two layer film are summarized in table 4. Again, the commercial film "Petalo Blue" was used as additional comparison.

**Table 4**

| Designation | Embossing + temper. | Rating touch | Re-wet [g] | Strike through [s] |
|---|---|---|---|---|
| 3a | No | 124 | 0.075 | 4.5 |
| 3b | yes, 35 °C | 180 | 0.077 | 4.25 |
| 3c | yes, 60 °C | 216 | 0.313 | 4.34 |
| 3d (Comp.) | yes, 80 °C | 145 | 0.401 | 5.83 |
| Petalo Blue (Comp.) | | 127 | 0.45 | 3.4 |

In this example the influence of different embossing temperatures was evaluated. Without embossing a top sheet with approximately the same haptic and strike through, but much better re-wet is obtained as compared to the commercial standard. With embossing according to the invention the haptic properties are considerably improved. The actual embossing temperature has to be chosen as compromise between improving haptics and losses with respect to the hygienic properties. It can be seen that the re-wet increases at higher embossing temperature, which is due to deformation of the perforation shape. An embossing at too high temperatures leads to inferior re-wet and strike through and also worse haptic properties.

Overall the examples 1 to 3 show that the films according to the invention with a top layer having inert particles in it have an improved touch. For a given film it is possible to obtain excellent touch together with very good hygienic properties, especially re-wet and strike through. An embossing step is easy to perform and will not need costly apparatus or processing control as compared to e.g. use of micro apertures in EP 1621169.

## Claims

1. Perforated multilayer film having a top layer comprising a thermoplastic material and from 5 to 25 % by weight of the top layer of inert particles with a mean diameter of 1 to 5 µm and an aspect ratio from 1 to 5.

2. Film according to claim 1, **characterized in that** the thermoplastic material is chosen among low density polyethylenes, linear low density polyethylenes and mixtures of one or more low density polyethylenes and/or linear low density polyethylenes.

3. Film according to claim 1 or 2, **characterized in that** the inert particles are chosen from calcium carbonate, glass beads, barium sulfate or mixtures thereof.

4. Film according to any one of claims 1 to 3, **characterized in that** the top layer has a textured surface.

5. Film according to any one of claims 1 to 4, **characterized in that** it has been embossed at temperatures below 70 °C, preferably lower than 60 °C, especially preferred lower than 50 °C, after perforating.

6. Film according to claim 5, **characterized in that** the embossing pattern is chosen among dots, interrupted and continuous lines.

7. Film according to claim 6, **characterized in that** the embossing pattern is straight or curved, crossing or staggered lines, preferably straight, rectangular crossing lines.

8. Film according to claim 7, **characterized in that** the distance between the lines is 100 to 800 µm, preferably 200 to 600 µm and especially preferred 300 to 500 µm.

9. Film according to any one of claims 1 to 8, **characterized in that** the form of the perforations is chosen among simple convex polygons, especially rhombus, trapeze, rectangle, hexagon, pentagon, heptagon and octagon, and ellipses or circles.

10. Film according to any one of claims 1 to 9, **characterized in that** the size of one perforation ranges from 0.1 to 10 mm², preferably from 0.2 to 7 mm² and especially from 2 to 4 mm² with distances between the perforations from 0.1 mm to 1 mm, preferably from 0.3 to 0.5 mm.

11. Method for manufacturing films according to any one of claims 1 to 10, comprising the steps of
a) providing multilayer precursor film
b) perforating the film
**characterized by**
c) embossing the perforated film at a temperature of lower than 70 °C.

12. Method according to claim 11, **characterized in that** the multilayer film comprises a top layer comprising a thermoplastic material and from 5 to 25 % by weight of the top layer of inert particles with a mean diameter in the range from 1 to 5 µm.

13. Method according to claim 11 or 12, **characterized in that** the multilayer film is coextruded.

14. Method according to any one of claims 11 to 13, **characterized in that** the surface of the multilayer film is textured.

15. Method according to any one of claims 11 to 13, **characterized in that** the film is printed, stretched, and/or ring-rolled.

16. Absorbent article, **characterized in that** the topsheet is a multilayer perforated film according to any one of claims 1 to 10 whereby the top layer forms the body facing side.

17. Article according to claim 16, **characterized in that** it is a diaper, catamenial pad or adult incontinence article.
